# EUROPEAN PATENT APPLICATION

(11) **EP 2 837 614 A1**
(43) Date of publication of application: **18.02.2015**
(21) Application number: 13180456.9
(22) Date of filing: 14.08.2013
(51) Int. Cl.: C07C 29/60, C07C 31/20

(54) **Process for the manufacture of propanediol**

(71) Applicant: Solvay SA, 1120 Bruxelles (BE)
(72) Inventor: Su, Fangzheng, 1030 Brussels (BE)
(74) Representative: Vande Gucht, Anne

(57) **Abstract**

A process for manufacturing 1,3-propanediol by reacting glycerol with hydrogen in the presence of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium.

## Description

The invention relates to a process for the manufacture of 1,3-propanediol, in particular for the manufacture of 1,3-propanediol by hydrogenation of glycerol.

Trimethylene glycol (1,3-propanediol) is mainly used as a building block in the production of polymers such as polytrimethylene terephthalate and it can be formulated into a variety of industrial products including composites, adhesives, laminates, coatings, moldings, aliphatic polyesters and copolyesters. It can also be used as a solvent, an ingredient for a food composition, an antifreeze and a wood paint.

Trimethylene glycol may be chemically synthesized by the hydration of acrolein, by the hydroformylation of ethylene oxide to afford 3-hydroxypropionaldehyde, which is hydrogenated to give 1,3-propanediol, by bioprocessing of glucose and glycerol by certain micro-organisms, or by catalytic hydrogenation of glycerol.

Arundhathi et al. (ChemSusChem Communications, Article First published on line 21 June 2013) discloses the hydrogenolysis of glycerol to 1,3-propanediol over alumina supported platinum tungsten catalysts. Mixtures of propanediols and propanols are obtained, a large amount of water is used as solvent and yields of 1,3-propanediol are generally not satisfactory.

Opportunities remain therefore to further improve the 1,3-propanediol yield in the processes disclosed in the prior art. This and other problems are solved by the present invention described below.

In a first embodiment, the invention is related to a process for manufacturing 1,3-propanediol by reacting glycerol with hydrogen in the presence of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium.

One of the essential features of the present invention is that the reaction is carried out in the presence of a limited amount of water. This allows to lessen the use of water without affecting negatively the 1,3-propanediol selectivity and consequently to improve the process volumic productivity.

In a second embodiment, the invention relates to a process for making a polyester comprising obtaining 1,3-propanediol according to the process of the first embodiment and further submitting said 1,3-propanediol to a reaction with a carboxylic acid and/or a carboxylic acid ester.

In a third embodiment, the invention relates to a process for making a polyester fiber comprising obtaining a polyester according to the process of the second embodiment and further converting said polyester in to a fiber.

In the process according to the first embodiment of the invention, the liquid medium may be a single-phase or multi-phase medium.

The liquid medium is composed of all of the dissolved or dispersed solid compounds, dissolved or dispersed liquid compounds and dissolved or dispersed gaseous compounds at the temperature of the reaction.

The liquid medium comprises the reactants, the catalyst, the solvent if any, the impurities present in the reactants, in the solvent if any and in the catalyst, the reaction intermediates, the product and the by-products of the reaction.

The reactants are the glycerol and the hydrogen. The product is 1,3-propanediol. The catalyst, the reaction intermediates, the by-products and the solvent are as described here below.

In the process according to the first embodiment of the invention, the reaction is carried out in a liquid medium containing containing less than 900 g of water per kg of liquid medium. The content of water in the liquid reaction medium is preferably lower than or equal to 800 g/kg, more preferably lower than or equal to 600 g/kg, yet more preferably lower than or equal to 400 g/kg, still more preferably lower than or equal to 200 g/kg and most preferably lower than or equal to 100 g/kg. That content of water is higher than or equal to 3 g/kg of liquid medium, preferably higher than or equal to 5 g/kg, more preferably higher than or equal to 8 g/kg, yet more preferably higher than or equal to 10 g/kg, still more preferably higher than or equal to 50 g/kg, most preferably higher than or equal to 75 g/kg, yet most preferably higher than or equal to 90g/kg and still most preferably higher than or equal to 95 g/kg.

Water can be water generated by the hydrogenation reaction or external water e.g. water present in the reactants, the catalyst, the solvent if any or added water.

In the process according to the first embodiment of the invention, the glycerol can be synthetic glycerol or natural glycerol or any mixture thereof. Synthetic glycerol is glycerol which has been obtained from non renewable raw materials. The glycerol is preferably natural glycerol i.e. glycerol which has been prepared in a conversion process of renewable raw materials. By glycerol which has been prepared in a conversion process of renewable raw materials one intends to denote glycerol obtained in a process selected from the group consisting of hydrolysis, saponification, transesterification, aminolysis and hydrogenation of oils and/or fats of animal and/or plant and/or algae origin, fermentation, hydrogenation and hydrogenolysis of mono- and polysaccharides and derived alcohols, derived from or occurring naturally in the biomass, and any combination thereof. Glycerol which has been obtained during the manufacture of biodiesel, i.e. during the transesterification of oils and/or fats of animal and/or plant, and preferably during the transesterification of oils and/or fats of plant origin, is particularly convenient. Glycerol which has been obtained in the manufacture of biodiesel is more particularly convenient.

In the process according to the first embodiment of the invention, the hydrogen can be obtained from any source. The hydrogen is preferably molecular hydrogen.

In the process according to the first embodiment of the invention, the hydrogen is preferably obtained from at least one process selected from the group consisting of steam reforming of hydrocarbons, partial oxidation of hydrocarbons, autothermal reforming of hydrocarbons, water-gas shift, coal gasification, pyrolysis of organic waste products (tar, lignite pitch, petroleum distillation residues, plastics, rubber, cellulose, paper, textile, wood, straw, mixed municipal waste...) and co-pyrolysis of organic wastes products with coal (including bituminous coal, lignite,...), thermal and non-thermal plasma cracking of mixtures of water or steam, and fuels, biomass gasification, biomass pyrolysis and subsequent gasification, thermal or catalytic decomposition of nitrogen compounds like ammonia, hydrazine, biochemical hydrogen fermentation, enzymatic treatment of natural sugars (e.g. xylose), steam reforming of alcohols, for instance monoalcohols, such methanol or ethanol, and polyols, such as propanediols and glycerine, alkaline cracking of insaturated fatty acid particularly oleic and ricinoleic acid, electrolysis of an aqueous solution of a hydrogen halide, electrolysis of an aqueous solution of a metal halide like for instance sodium chloride or potassium chloride, hydrolysis of metals or metal hydrides, water splitting from for instance alkaline electrolysis, proton-exchange membrane electrolysis, solid oxide electrolysis, high pressure electrolysis, high temperature electrolysis, photoelectrochemical water splitting, photocatalytic water splitting, photobiological water splitting and water thermolysis. When the selected process is electrolysis of an aqueous solution of a hydrogen halide, the hydrogen halide is often selected from hydrogen chloride, hydrogen fluoride and any mixture thereof, and is frequently hydrogen chloride. When the selected process is electrolysis of an aqueous solution of sodium chloride or potassium chloride, electrolysis can be any of mercury electrolysis, membrane electrolysis or diaphragm electrolysis. Membrane electrolysis is preferred.

In the process according to the first embodiment of the invention, the hydrogen can be used in admixture with another compound. The other compound is usually selected from the group consisting of nitrogen, helium, argon, carbon dioxide, steam, saturated hydrocarbons, and any mixture thereof. In the process according to the first embodiment of the invention, the resulting mixture containing hydrogen comprises generally at least 10 % by volume of hydrogen, usually at least 50 % by volume, preferably at least 75 % by vol, more preferably at least 90 % by volume, still more preferably at least 95 % by volume, yet more preferably at least 99 % by volume and most preferably at least 99.9 % by volume. That mixture comprises generally at most 99.99 % of hydrogen by volume. A mixture which consists essentially of hydrogen is also convenient. A mixture which consists of hydrogen is also suitable.

In the process according to the first embodiment of the invention, by alumina, one intends to denote an aluminum hydroxide, an aluminum oxide, any compound resulting from a thermal treatment, especially from a hydrothermal treatment, of aluminum hydroxide, and any mixture thereof, as defined in Ullmann's Encyclopedia of Industrial Chemistry, http://onlinelibrary.wiley.com/doi/10.1002/14356007.a01_557/pdfL.K. Hudson et al., published on line 15 JUN 2000, pages 607-645. Aluminum trihydroxides, aluminum oxide hydroxides and pseudoboehmite are examples of an aluminum hydroxide. Gibbsite, Bayerite or Nordstrandite are examples of an aluminum trihydroxide. Boehmite or Diaspore are examples of an aluminum oxide hydroxide. Corundum is an example of an aluminum oxide. Chi-, kappa-, gamma-, delta-, theta-, and eta-alumina are examples of compound resulting from a thermal treatment, especially an hydrothermal treatment, of an aluminum hydroxide.

In the process according to the first embodiment of the invention, the alumina in the supported catalyst is preferably selected from chi-, kappa-, gamma-, delta-, theta-, eta-alumina, and any mixture thereof, more preferably from gamma-, delta-, theta-alumina, and any mixture thereof, yet more preferably from gamma alumina and most preferably from delta-, theta-alumina and any mixture thereof. An alumina in the supported catalyst containing less than 10 percent by weight of gamma-alumina is also convenient.

In the process according to the first embodiment of the invention, the alumina can be crystalline, amorphous or a mixture thereof, according to X-ray diffraction analysis. The alumina is preferably a mixture of crystalline and amorphous alumina. X-ray diffraction patterns of crystalline alumina can be found in http://www.sasoltechdata.com/tds/PURALOX_CATALOX.pdf.

In the process according to the first embodiment of the invention, the supported catalyst, preferably exhibits at least one of the following features:
- a nitrogen BET specific area higher than or equal to 50 m²/g and lower than or equal to 400 m²/g;
- a nitrogen BET total pore volume higher than or equal to 0.2 cm³/g and lower than or equal to 1.5 cm³/g;
- a nitrogen BET average pore size higher than or equal to 2 nm and lower than or equal to 100 nm;
- a TEM average particle size of the first compound lower than or equal to 15 nm;
- a content of the at least one first compound with respect to the catalyst higher than or equal to 0.1 percent by weight and lower than or equal to 20 % by weight; and
- a content of the at least one second compound with respect to the catalyst higher than or equal to 1 percent by weight and lower than or equal to 30 percent by weight.

In the process according to the first embodiment of the invention, the at least one first compound of an element selected from iridium, rhodium, palladium and platinum, is more preferably selected from a compound palladium and platinum, and is most preferably a compound of platinum.

In the process according to the first embodiment of the invention, the at least one first compound content of the supported catalyst with respect to the catalyst is usually higher than or equal to 0.1 % by weight, preferably higher than or equal to 0.5 % by weight, more preferably higher than or equal to 1 % by weight, yet more preferably higher than or equal to 2 % by weight, and most preferably higher than or equal to 4% by weight. That at least one first compound content is usually lower than or equal to 20 % by weight, preferably lower than or equal to 15% by weight, more preferably lower than or equal to 10% by weight, yet more preferably lower than or equal to 8% by weight and most preferably lower than or equal to 6% by weight. When more than one first compound is present, the above mentioned content applies to the sum of the first compounds. The weight percent are expressed in weight of elemental iridium (Ir), rhodium (Rh), palladium (Pd) and/or platinum (Pt) per weight of catalyst. These features are especially suited when the at least one first compound is a compound of platinum. A catalyst with a content of about 5 weight percent of the at least one first compound, preferably a compound of platinum, is particularly convenient.

In the process according to the first embodiment of the invention, the at least one second compound of an element selected from chromium, molybdenum and tungsten, more preferably selected from compounds of molybdenum and tungsten, and is most preferably a compound of tungsten.

In the process according to the first embodiment of the invention, the at least one second compound content of the supported catalyst with respect to the catalyst is usually higher than or equal to 1 % by weight, preferably higher than or equal to 2 % by weight, more preferably higher than or equal to 5 % by weight, yet more preferably higher than or equal to 8 % by weight, and most preferably higher than or equal to 9% by weight. That at least one second compound content is usually lower than or equal to 30% by weight, preferably lower than or equal to 25% by weight, more preferably lower than or equal to 20% by weight, yet more preferably lower than or equal to 15% by weight and most preferably lower than or equal to 11 % by weight. When more than one second compound is present, the above mentioned content applies to the sum of the second compounds. The weight percent are expressed in weight of chromium trioxide (CrO₃), molybdenum trioxide (MoO₃) and/or tungsten trioxide (WO₃) per weight of catalyst. These features are especially suited when the at least one second compound is a compound of tungsten. A catalyst with a content of about 10 weight percent of the at least one second compound, preferably a compound of tungsten, is particularly convenient.

In the process according to the first embodiment of the invention, at least one part of the at least one first compound in the supported catalyst is preferably present in the metallic form. The ratio between the at least one first compound present under metallic form and the total amount of the at least one first compound is preferably higher than or equal to 5 %, more preferably higher than or equal to 10 %, still more preferably higher than or equal to 50 %, yet preferably higher than or equal to 90 %, most preferably higher than or equal to 95 %, and yet most preferably higher then or equal to 99 %. The weight percent are expressed as above for the at least one first compound. A catalyst where the at least one first compound is essentially under metallic form is particularly convenient. These features are especially suited when the at least one first compound is a compound of platinum.

In the process according to the first embodiment of the invention, at least one part of the at least one second compound in the supported catalyst is preferably present as an oxide. The ratio between the at least one second compound present under oxide form and the total amount of the at least one second compound is preferably higher than or equal to 5 %, more preferably higher than or equal to 10 %, still more preferably higher than or equal to 50 %, yet preferably higher than or equal to 90 %, most preferably higher than or equal to 95 %, and yet most preferably higher than or equal to 99 %. The weight percent are expressed as above for the at least one second compound. A catalyst where the at least one second compound is essentially under oxide form is particularly convenient. These features are especially suited when the at least one second compound is a compound of tungsten, in which case tungsten is preferably present as tungsten trioxide, WO₃.

In the process according to the first embodiment of the invention, the at least one first compound is a compound of platinum, preferably platinum metal, and the at least one second compound is a compound of tungsten, preferably tungsten trioxide.

In the process according to the first embodiment of the invention, the supported catalyst preferably contains less than 10 weight percent of titanium expressed as titanium dioxide.

In the process according to the first embodiment of the invention, the catalyst is usually in a non-powder form selected from the group consisting of rings, beads, spheres, saddles, pellets, tablets, extrudates, granules, crushed, flakes, honeycombs, filaments, cylinders, polygons and any mixture thereof, or in powder form. The preferred form depends usually of the type of reactor used. A powder form is preferred when a slurry or fluidized-bed reactor is used for carrying out the reaction, while non-powder forms are preferred when a fixed bed reactor or a trickle bed reactor is used for carrying out the reaction.

In the process according to the first embodiment of the invention, the reaction may be carried out in the absence or in the presence of a solvent. The solvent may be selected from the group consisting of inert inorganic solvent, inert organic solvent, and combinations thereof. Examples of inert inorganic solvents are water, supercritical carbon dioxide, and inorganic ionic liquids. Examples of inert organic solvents are alcohols, ethers, saturated hydrocarbons, esters, perfluorinated hydrocarbons, nitriles, amides and any mixture thereof. Examples of alcohols are methanol, ethanol, 1-propanol, 2-propanol, ethylene glycol, 1,2-propanediol, 1,3-propanediol. Examples of ethers are diethylene glycol, dioxane, tetrahydrofuran, ethylene glycol monomethyl ether, ethylene glycol monoethyl ether, ethylene glycol dimethyl ether, diethylene glycol monomethyl ether and diethylene glycol dimethyl ether. An example of saturated hydrocarbons is cyclohexane. An example of esters is ethyl acetate. Examples of perfluorinated hydrocarbons are perfluorinated alkane such as perfluorinated hexane, heptane, octane, nonane, cyclohexane, methylcyclohexane, dimethylcyclohexane or trimethylcyclohexane, perfluoroethers like Galden® HT from Solvay Solexis, perfluorotetrahydrofurane or perfluoroamine like FluorinertTM FC from 3M. An example of nitriles is acetonitrile. An example of amides is dimethylformamide.

In the process according to the first embodiment of the invention, when the reaction is carried out in the presence of a solvent, different from water, the content of the solvent in the liquid reaction medium is usually higher than or equal to 1 g/kg of liquid reaction medium, preferably higher than or equal to 2 g/kg, more preferably higher than or equal to 5 g/kg, yet more preferably higher than or equal to 10 g/kg, still more preferably higher than or equal to 50 g/kg, most preferably higher than or equal to 100 g/kg, yet most preferably higher than or equal to 150g/kg and still most preferably higher than or equal to 200g/kg. That content of solvent is usually lower than or equal to 999 g/kg, preferably lower than or equal to 950 g/kg, more preferably lower than or equal to 900 g/kg, yet more preferably lower than or equal to 850 g/kg, still more preferably lower than or equal to 825g/kg and most preferably lower than or equal to 800 g/kg.

In the process according to the first embodiment of the invention, the reaction may also be carried out solvent-less, i.e. for a content of the solvent, preferably different from water, in the liquid reaction medium lower than 1 g/kg.

The process according to the first embodiment of the invention can be carried out according to any mode of operation. The mode of operation can be continuous or discontinuous. By continuous mode of operation, one intends to denote a mode of operation where the glycerol and hydrogen are added continuously in the process, and the 1,3-propanediol is continuously withdrawn from the process. Any other mode of operation is considered as discontinuous.

In the process according to the first embodiment of the invention, the reaction is carried out in a liquid reaction medium.

The process according to the first embodiment of the invention can be carried out in reaction apparatuses made of or coated with materials which are suitable for hydrogenation under pressure, resistant in the presence of corrosive compounds under the reaction conditions. Suitable materials can be selected from the group consisting of glass, enamel, enameled steel, graphite, impregnated graphite, like for example graphite impregnated with a perfluorinated polymer such as polytetrafluoroethylene, or graphite impregnated with a phenolic resin, polyolefins, like for instance polyethylene or polypropylene, fluorinated polymers, like perfluorinated polymers such as for instance polytetrafluoroethylene, poly(perfluoropropylvinylether), copolymers of tetrafluoroethylene and hexafluoropropylene, and like partially fluorinated polymers such as for instance poly(vinylidene fluoride), polymers comprising sulphur, like for instance polysulphones or polysulphides, metals, like for instance tantalum, titanium, copper, gold, silver, nickel and molybdenum, and metal alloys, like for instance alloys containing nickel, such as Hastelloy B, Hastelloy C, alloys containing molybdenum, such as Inconel 600, Inconel 625 or Incoloy 825.

The materials may be used within the mass, or in the form of cladding, or else by means of any coating process. Enameled steel is particularly convenient. Glass-lined apparatuses are also convenient.

In the process according to the first embodiment of the invention, the reaction is carried out at a temperature preferably higher than or equal to 70°C, more preferably higher than or equal to 80°C, yet more preferably higher than or equal to 90 °C and most preferably higher than or equal to 100°C. That temperature is preferably lower than or equal to 300°C, more preferably lower than or equal to 200°C, yet more preferably lower than or equal to 180°C and most preferably lower than or equal to 150°C.

In the process according to the first embodiment of the invention, the reaction is preferably carried out at a hydrogen partial pressure preferably higher than or equal to 1 bar absolute (1 bara), more preferably higher than or equal to 5 bara and most preferably higher than or equal to 10 bara. That hydrogen partial pressure is preferably lower than equal to 200 bara, more preferably lower than equal to 150 bara and most preferably lower than equal to 120 bara.

In the process according to the first embodiment of the invention carried out under continuous mode, the residence time which is the ratio of the volume of the liquid medium to the flow rate by volume of the reactants, depends on the reaction rate, on the hydrogen partial pressure, on the temperature, on the thoroughness with which the liquid medium is mixed, and on the activity and concentration of the supported catalyst. This residence time is usually higher than or equal to 5 minutes, often higher than or equal to 15 minutes, frequently higher than or equal to 30 minutes and in particular higher than or equal to 60 minutes. This residence time is usually lower than or equal to 25 hours, often lower than or equal to 20 hours, frequently lower than or equal to 10 and in particular lower than or equal to 5 hours.

In the process according to the first embodiment of the invention carried out under continuous mode, especially when a gas and a liquid phase are present, the residence time for the liquid phase, which is the ratio of the volume of the reactor volume to the flow rate by volume of the liquid phase, is usually higher than or equal to 5 minutes, often higher than or equal to 15 minutes, frequently higher than or equal to 30 minutes and in particular higher than or equal to 60 minutes. This residence time is usually lower than or equal to 25 hours, often lower than or equal to 10 hours and frequently lower than or equal to 5 hours.

In the process according to the first embodiment of the invention carried out under continuous mode, especially when a gas and a liquid phase are present, the residence time for the gas phase, which is the ratio of the volume of the reactor volume to the flow rate by volume of the gas phase, is usually higher than or equal to 1 second, often higher than or equal to 5 seconds, frequently higher than or equal to 10 seconds and in particular higher than or equal to 30 seconds. This residence time is usually lower than or equal to 10 minutes, often lower than or equal to 5 minutes and frequently lower than or equal to 2 minutes.

In the process according to the first embodiment of the invention carried out under discontinuous mode, the reaction time required for the process according to the invention depends on the reaction rate, on the hydrogen partial pressure, on the temperature, on the thoroughness with which the reaction mixture is mixed, and on the activity and concentration of the supported catalyst. The required reaction time is usually higher than or equal to 5 minutes, often higher than or equal to 15 minutes, frequently higher than or equal to 30 minutes, in particular higher than or equal to 60 minutes, and more specifically higher than or equal to 160 min. This residence time is usually lower than or equal to 25 hours, often lower than or equal to 20 hours, frequently lower than or equal to 10 and in particular lower than or equal to 5 hours.

In the process according to the first embodiment of the invention carried out under discontinuous mode, especially when a gas and a liquid phase are present, the reaction time for the liquid phase is usually higher than or equal to 5 minutes, often higher than or equal to 15 minutes, frequently higher than or equal to 30 minutes, in particular higher than or equal to 60 minutes, and more specifically higher than or equal to 180 min. This reaction time is usually lower than or equal to 25 hours, often lower than or equal to 10 hours and frequently lower than or equal to 5 hours.

In the process according to the first embodiment of the invention carried out under discontinuous mode, especially when a gas and a liquid phase are present, the reaction time for the gas phase is usually higher than or equal to 1 second, often higher than or equal to 5 seconds, frequently higher than or equal to 10 seconds and in particular higher than or equal to 30 seconds. This reaction time is usually lower than or equal to 10 hours, often lower than or equal to 5 hours and frequently lower than or equal to 2 hours.

In the process according to the first embodiment of the invention, especially when the reaction is carried out continuously, the molar ratio between the flow rates of hydrogen and glycerol is usually higher than or equal to 0.1 mol/mol, often higher than or equal to 0.5 mol/mol, frequently higher than or equal to 0.7 mol/mol and in particular higher than 1 mol/mol. This ratio is usually lower than or equal to 100 mol/mol, often lower than or equal to 50 mol/mol, frequently lower than or equal to 20 mol/mol and in particular lower than or equal to 10 mol/mol.

The process according to the first embodiment of the invention may be carried out in any type of reactor. The reactor, especially when the reaction is carried out in the presence of a catalyst, may be selected from the group consisting of a slurry reactor, a fixed bed reactor, a trickle bed reactor, a fluidized bed reactor, and combinations thereof. A slurry reactor or a trickle bed reactor is particularly convenient. A trickle bed reactor is more particularly suitable. A trickle reactor fed at co-current by hydrogen and the glycerol is very particularly convenient. One or more reactors could be used.

In a simple way of carrying out the process according to the first embodiment of the invention, the process can be carried out discontinuously, in the following manner an autoclave which is provided with a stirring or mixing unit and which can be thermostated is charged, in a suitable manner, with glycerol to be hydrogenated, the catalyst and a possible solvent. Thereafter, hydrogen is forced in until the desired pressure is reached, and the mixture is heated to the chosen reaction temperature while mixing thoroughly. The course of the reaction can be readily monitored by measuring the amount of hydrogen consumed, which is compensated by feeding in further hydrogen until the targeted conversion for glycerol is reached.

The mixture present after the hydrogenation can be worked up, for example, as follows : when the hydrogenation is complete, the reaction vessel is cooled, the pressure is let down and the catalyst is filtered off and rinsed with the possible solvent used, and the possible solvent used is then removed under reduced pressure or under atmospheric pressure. The crude product which remains can likewise be purified further by distillation under reduced pressure or under atmospheric pressure. When high-boiling solvents are used, it is also possible first to distil off the propanediol. When no solvent is used the mixture can be submitted directly to a distillation under reduced pressure or under atmospheric pressure. The recovered catalyst e.g by filtration can be reused in the discontinuous process as such or after reactivation by a physico-chemical treatment.

In another way of carrying out the process according to the first embodiment of the invention, the process can be carried out continuously, in the following manner: a vertical cylindrical reactor which can be thermostated is charged with the catalyst provided in a suitable shape in order to obtain a fixed bed of catalyst in the reactor. The reactor is fitted on its top part with inlet ports to feed the glycerol to be hydrogenated, neat or dissolved in a solvent and hydrogen, on its bottom part with an outlet port to recover the reaction mixture, with a regulation pressure device, and a vessel, to recover the reaction mixture and separate the liquid phase and the gas phase. Thereafter, hydrogen is introduced in the reactor until the desired pressure is reached, then the reactor is heated to the chosen reaction temperature, hydrogen and the glycerol, neat or dissolved in a solvent, are forced continuously in the reactor at the selected molar ratio and the reaction mixture is collected continuously in the recovery vessel. The extent of the reaction can be readily monitored by analyzing the composition of the liquid separated in the collection vessel. The liquid mixture present in the recovery vessel can be worked up, for example, by distillation under reduced pressure or under atmospheric pressure.

When the process according to the first embodiment of the invention is carried out continuously, a continuous process purge of gaseous by-products of the reaction or of gaseous contaminants of raw materials may be present and the main part of the non reacted hydrogen can be recycled to the reactor.

Gas chromatography is usually used for assessing the content of the organic compounds in samples withdrawn at various stages of the process.

The present invention also relates in a second embodiment to a process for making a polymer selected from the group consisting of a polyether, a polyurethane, a polyester, and any mixture thereof, comprising obtaining 1,3-propanediol by reacting glycerol with hydrogen in the presence of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium, and further using said 1,3-propanediol as raw materials.

The process for making the polyether comprises obtaining 1,3-propanediol by reacting glycerol with hydrogen in the presence of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium, and further submitting said 1,3-propanediol to a reaction with at least one compound selected from the group consisting of a halogenated organic compound, an organic epoxide, an alcohol, or any mixture thereof.

The process for making the polyurethane comprises obtaining 1,3-propanediol by reacting glycerol with hydrogen in the presence of of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium, and further submitting said 1,3- propanediol to a reaction with a polyisocyanate, preferably a diisocyanate.

In the process for making the polymer according to the invention, the polymer is preferably a polyester.

The present invention therefore also relates to a process for making a polyester comprising obtaining 1,3-propanediol by reacting glycerol with hydrogen in the presence of of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium, and further submitting said 1,3-propanediol to a reaction with a carboxylic acid and/or a carboxylic acid ester.

The features mentioned above for the process for manufacturing the 1,3-propanediol are applicable for obtaining the 1,3-propanediol used for making the polymer, preferably the polyester.

In the process for making a polyester according to the invention, the carboxylic acid is preferably a polycarboxylic acid, more preferably a dicarboxylic acid. The polycarboxylic acid is preferably selected from the group consisting of an aliphatic saturated or unsaturated, an aromatic, an alkylaromatic saturated or unsaturated, a heteroaromatic, an alkylheteroaromatic saturated or unsaturated, or any mixture thereof.

The preferred aliphatic dicarboxylic acid contains from 2 to 16 carbon atoms are more preferably selected from the group consisting of oxalic acid, malonic acid, succinic acid, glutaric acid, adipic acid, sebacic acid, azelaic acid and any mixture thereof.

The preferred unsaturated dicarboxylic acid is selected from the group consisting of fumaric acid, maleic acid, and any mixture thereof.

The preferred aromatic is selected from the group consisting of o-phthalic acid, m-phthalic acid, p-phthalic acid (terephthalic acid), naphthalene dicarboxylic acid, and any mixture thereof. The preferred aromatic carboxylic acid is more preferably terephthalic acid.

The preferred alkyl aromatic is selected from the group consisting of 4-methylphthalic acid, 4-methylphthalic acid, and any mixture thereof.

The preferred unsaturated dicarboxylic aromatic acid is selected from the group consisting of vinyl phthalic acids, and any mixture thereof.

The preferred heteroaromatic acid is selected from the group consisting of furano dicarboxylic acids, and any mixture thereof, and is preferably 2,5-furano dicarboxylic acid.

In the process for making a polyester according to the invention, the carboxylic acid ester is preferably an ester of the above cited carboxylic acid, preferably a methyl, or ethyl ester. The preferred esters is selected from the group consisting of an ester of terephthalic acid, an ester of furano dicarboxylic acid, and any mixture thereof. The ester is more preferably a terephthalic acid ester, and most preferably dimethyl terephthalate.

The production of the polyester is described, for example, in Ullmann's Encyclopedia of Industrial Chemistry, Horst Köpnick, Manfred Schmidt, Wilhelm Brügging, Jörn Rüter and Walter Kaminsky, Published Online : 15 JUNE 2000, DOI : 10.1002/14356007.a21_227, pages 623-649.

The polymer, preferable a polyester, obtained according to the process of the invention usually exhibits a ¹⁴C/¹²C higher than or equal to 0.33 10⁻¹², often higher than or equal to 0.5 10⁻¹², frequently higher than or equal to 0.75 10⁻¹², in many case higher than or equal to 1.0 10⁻¹² and in particular higher than or equal to 1.1 10⁻¹²_{.}

In a further embodiment, the invention also relates to a polyester exhibiting a ¹⁴C/¹²C higher than or equal to 0.33 10⁻¹², often higher than or equal to 0.5 10⁻¹², frequently higher than or equal to 0.75 10⁻¹², in many case higher than or equal to 1.0 10⁻¹² and in particular higher than or equal to 1.1 10⁻¹².

The polyester is preferably obtainable by reacting 1,3-propanediol obtained by reacting glycerol with hydrogen in the presence of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium, and further submitting said 1,3-propanediol to a reaction with a carboxylic acid and/or a carboxylic acid ester, as described here above.

The polyester is more preferably obtained by reacting 1,3-propanediol obtained by reacting glycerol with hydrogen in the presence of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium, and further submitting said propanediol to a reaction with a carboxylic acid and/or a carboxylic acid ester, as described here above.

The present invention also relates in a third embodiment to a process for making a polyester fiber comprising obtaining 1,3-propanediol by reacting 1,3-propanediol obtained by reacting glycerol with hydrogen in the presence of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3 g and less than 900 g of water per kg of liquid medium , further submitting said 1,3-propanediol to a reaction with a carboxylic acid and/or a carboxylic acid ester to obtain a polyester and further converting said polyester into a fiber.

The methods for measuring the ¹⁴C content are precisely described in standards ASTM D 6866 (notably D 6866-06, D 6866-08 and D 6866-12) and in standards ASTM 7026 (notably D 7026-04). The method preferably used is described in standard ASTM D6866-12.

The features mentioned above for the process for manufacturing the 1,3-propanediol and for the process for making the polyester are applicable for obtaining the 1,3-propanediol and the polyester used for making the polyester fiber.

The production of the polyester fibers is described, for example, in Ullmann's Encyclopedia of Industrial Chemistry, Helmut Sattler and Michael Schweizer, Published Online : 15 OCT 2011, DOI : 10.1002/14356007.o10_o01, pages 1 to 34.

The polyester fibers have numerous applications and can be used in tires, rope, cordage, sewing thread, seat belts, hoses, webbing, coated fabrics, carpets, apparel, home fashions, upholstery, medical, interlinings, filtration, fiberfill, high-loft, roofing, geotextiles, and substrates, for instance.

In a fourth embodiment, the invention also relates to a process for preparing a supported catalyst comprising at least one first compound of an element selected from iridium, rhodium, palladium and platinum and at least one second compound of an element selected from chromium, molybdenum and tungsten, both compounds being supported on alumina, comprising the following steps: (I) impregnating an alumina with a precursor of the at least one second compound, (II) drying the impregnated alumina of step (I), (III) calcining the dried alumina of step (II), (IV to VI) repeating the steps (I) to (III) with a precursor of the at least one first compound and (VII) treating with hydrogen the calcined alumina of step (VI).

All the features disclosed here above for the first and second compound supported on the alumina are applicable to the process for preparing the catalyst.

The impregnation of step (I) can be carried out according to any know technique of impregnation as described in "Applied Catalysis A: General, 1995, 133, 281-292". Successive wet-impregnation is preferred. Any solvent can be used provided that the first and second compounds precursors are soluble therein. Water is a preferred solvent. Any first and second compounds precursors can be used provided that they are soluble in the solvent used. When the first compound is platinum and the second compound is tungsten, chloroplatinic acid and ammonium metatungstate are preferred, in particular when the solvent used is water.

The drying of steps (II) and (V) can be carried out according to any known technique. Drying is carried out at a temperature preferably higher than or equal to 50°C, more preferably higher than or equal to 70°C, and most preferably higher than or equal to 90°C. This drying temperature is preferably lower than or equal to 180°C, more preferably lower than or equal to 150°C, and most preferably lower than or equal to 130°C. Drying is carried out at a pressure preferably higher than or equal to 400 mbar absolute, more preferably higher than or equal to 600 mbara, and most preferably higher than or equal to 800 mbara. This drying pressure is preferably lower than or equal to 1800 mbara, more preferably lower than or equal to 1500 mbara, and most preferably lower than or equal to 1200 mbara.

The calcination of steps (III) and (VI) can be carried out according to any know technique. Calcination is carried out at a temperature preferably higher than or equal to 300°C, more preferably higher than or equal to 350°C, and most preferably higher than or equal to 400°C. This calcination temperature is preferably lower than or equal to 700°C, more preferably lower than or equal to 650°C, and most preferably lower than or equal to 600°C.

The treatment under hydrogen of step (VII) can be carried out at a temperature preferably higher than or equal to 150°C, more preferably higher than or equal to 160°C, and most preferably higher than or equal to 170°C. This treatment temperature is preferably lower than or equal to 400°C, more preferably lower than or equal to 350°C, and most preferably lower than or equal to 325°C.

The examples below are intended to illustrate the invention without, however, limiting it.

### 1. Catalyst preparation

The supported catalysts were prepared by the successive wet-impregnating method. The support was impregnated with an aqueous solution (5 g H₂O/g support) of ammonium metatungstate hydrate (Aldrich) at about 25 °C for about 30 minutes, under agitation. The water was removed by using rotary evaporator at 60 °C, at about 100 mbar and for about 30 minutes, and the resulting sample was dried overnight at 110 °C under static air under 1 bara. The dried sample was then calcined at 450 °C under static air, at 1 bara, for 3 hours. The calcined sample was slurried with an aqueous solution of chloroplatinic acid hydrate (Aldrich, ∼ 38% Pt basis) (5 g H₂O/g calcined sample). The water was removed as above, the sample containing platinum was dried at 110 °Cas above, calcined at 450 °C as above, and treated at 300 °C with flowing hydrogen (50 ml/min/g of support) for 3 hours. The following alumina based oxides have been used as supports: gamma-Al₂O₃ (Puralox TM 100/150, Puralox TH 100/150, Puralox SCFa-140), delta/theta- Al₂O₃ (Puralox TH 100/90), 9.7 wt% TiO₂/Al₂O₃ (Puralox TH 100/150 Ti10) were provided by Sasol. The weight ratio of Pt and WO₃ in the final Pt/WO3/Al2O3 catalysts was 5:10

The catalysts are identified in the following Table 1.

**Table 1**

| Catalyst n° | S_{BET} of support (m²/g) | Vₚ of support (cm³/g) | Alumina based supports |
|---|---|---|---|
| 1 | 150 | 0.8 | Puralox TM 100/150 |
| 2 | 150 | 1.1 | Puralox TH 100/150 |
| 3 | 140 | 0.5 | Puralox SCFa-140 |
| 4 | 90 | 1.2 | Puralox TH 100/90 |
| 5 | 135 | 1.0 | Puralox TH 100/150 Ti10 |

### 2. Catalyst evaluation

The hydrogenation of glycerol has been carried out in a glass vessel fitted in a 100 ml Hastelloy (C-22) autoclave.

The vessel was firstly charged with a magnetic stirrer, 0.3 g catalyst and 10 g mixture of glycerol (VWR, vegetal origin, 99.5 % containing 0.73 g/kg of water) and water (Milli-Q water) and it was then placed in the autoclave. Subsequently, The autoclave was sealed and purged with nitrogen (N₂, Air Product, 99.998%)) by pressurizing and depressurizing several times and then with hydrogen (H₂, Air Product, 99.9995%) by pressurizing and depressurizing several times, and then heated to the desired temperature of 160 °C. Meanwhile, the hydrogen pressure was increased to 60 bar and stirring was set at 750 rpm. The time zero for the reaction was defined as the time at which the stirring was started. During the reaction, the pressure was always maintained at 60 bar. After an appropriate reaction time, the reaction was stopped and the autoclave was cooled down. The liquid product was separated by using polypropylene filter (0.8 µm of porosity) and then analyzed by gas chromatography (GC).

The GC has been carried out under the following conditions. The sample has been dissolved in dimethylformamide. The GC has been has been performed by injecting the solution on a CP Wax 57 CB column (25m*0.25mm*0.2um) using an appropriate temperature program and flame ionisation detection. Quantification has been done using an internal standard (2-chlorotoluene) and relative response factors, determined using standard reference products. The specific GC conditions were as follows:
- Injector temperature: 250 °C
- Detector temperature: 280 °C
- Oven temperature: 60 °C (5 min) - 10 °C/min - 240 °C (5 min)
- Split flow: 60 ml/min
- Flow rate: 1 ml/min (constant)

The mass balance was calculated for each test and it was found higher than 95 % in most cases.

The conditions for carrying out examples 1 to 9 are summarized in Table 2.

The water content of the liquid reaction medium has been calculated at the beginning and at the end of the reaction from the water content of the glycerol, the amount of the water added and the amount of water produced by the reaction. These contents are identified as water initial and water final in table 3.

**Table 2**

| Example | Catalyst | Reaction | | | | |
|---|---|---|---|---|---|---|
| | N° | water (g) | glycerol (g) | T(°C) | P (bara) | Time (h) |
| | | | | | | |
| 1 | 1 | 6 | 4 | 160 | 60 | 6 |
| 2 | 2 | 9.5 | 0.5 | 160 | 60 | 6 |
| 3 | 2 | 9 | 1 | 160 | 60 | 6 |
| 4 | 2 | 8 | 2 | 160 | 60 | 6 |
| 5 | 2 | 6 | 4 | 160 | 60 | 6 |
| 6 | 2 | 2 | 8 | 160 | 60 | 6 |
| 7 | 2 | 0 | 10 | 160 | 60 | 6 |
| 8 | 3 | 6 | 4 | 160 | 60 | 6 |
| 9 | 4 | 6 | 4 | 160 | 60 | 6 |
| 10 | 5 | 6 | 4 | 160 | 60 | 6 |

Examples 2, 3 and 7 are not according to the invention, the other examples are according to the invention.

The results of the tests are summarized in Table 3 here below.

The glycerol conversion and the selectivity of the various products have been calculated as follows:
Conversion = 100 X [(number of mole of glycerol introduced - (sum of number of mole of 1,3-propanediol, 1,2-propanediol, 1-propanol and 2-propanol recovered at the end of reaction)/(number of mole of glycerol introduced)].
Selectivity product = 100 X [(number of mole of product recovered at the end of reaction/( sum of number of mole of 1,3-propanediol, 1,2-propanediol, 1-propanol and 2-propanol recovered at the end of reaction)].
The TON1 value in Table 3 is the ratio between the number of moles of converted glycerol and the number of moles of platinum in the catalyst used.
The TON2 value in Table 3 is the ratio between the number of moles of produced 1,3-propanediol and the number of mole of platinum in the catalyst used.

**Table 3**

| Example | Water initial (wt %) | Water final (wt %) | Glycerol | Product selectivity (% mol) | | | | TON1 | TON2 |
|---|---|---|---|---|---|---|---|---|---|
| | | | Conversion (%mol) | 1,3-propanediol | 1,2-propanediol | 1-propanol | 2-propanol | | |
| 1 | 60 | 61.1 | 11.3 | 61.7 | 9.3 | 20.9 | 8.1 | 63.8 | 39.4 |
| 2 | 95 | 95.6 | 44.9 | 65 | 4 | 20.9 | 10.1 | 31.7 | 20.6 |
| 3 | 90 | 91.1 | 42.2 | 62.4 | 3.3 | 24.7 | 9.6 | 59.6 | 37.1 |
| 4 | 80 | 81.7 | 32.8 | 61.6 | 4.2 | 24.5 | 9.6 | 92.7 | 57.1 |
| 5 | 60 | 62.3 | 21.6 | 60 | 5.7 | 25.3 | 8.9 | 122 | 73.2 |
| 6 | 20 | 22.1 | 9.9 | 55.3 | 8.5 | 28.4 | 7.8 | 112 | 61.9 |
| 7 | 0.073 | 0.240 | 0.7 | 43.4 | 34.7 | 22 | n.d. | 9.9 | 4.3 |
| 8 | 60 | 61.3 | 12.5 | 60.4 | 7.9 | 22.1 | 9.7 | 70.6 | 42.6 |
| 9 | 60 | 62.4 | 22.2 | 59.2 | 5.5 | 25 | 10.3 | 125 | 74 |
| 10 | 60 | 62.7 | 25.9 | 60.2 | 5.5 | 24.4 | 9.9 | 146 | 87.9 |

## Claims

1. A process for manufacturing 1,3-propanediol by reacting glycerol with hydrogen in the presence of a supported catalyst, the supported catalyst comprising at least one first compound of an element selected from iridium, rhodium, palladium and platinum and at least one second compound of an element selected from chromium, molybdenum and tungsten, both compounds being supported on alumina, the reaction being carried out in a liquid medium containing water in an amount of at least 3g and less than 900 g of water per kg of liquid medium.

2. The process according to claim 1, wherein the liquid medium contains at most 800 g of water per kg of liquid medium.

3. The process according to claim 2, wherein the liquid medium contains at most 600 g of water per kg of liquid medium.

4. The process according to claim 3, wherein the liquid medium contains at most 400 g of water per kg of liquid medium.

5. The process according to any one of claims 1 to 4, wherein the alumina in the supported catalyst is selected from gamma alumina, delta alumina, theta alumina, and any mixture thereof.

6. The process according to claim 5, wherein the alumina in the supported catalyst is gamma alumina.

7. The process according to claim 5, wherein the alumina in the supported catalyst is selected from delta alumina, theta alumina, and any mixture thereof.

8. The process according to any one of claims 1 to 7, wherein in the supported catalyst at least part of the first compound is present under metallic form and at least part of the second compound is present under oxide form.

9. The process according to any one of claims 1 to 8, wherein in the supported catalyst the first compound is a compound of platinum and the second compound is a compound of tungsten.

10. The process according to any one of claims 1 to 9, wherein the supported catalyst contains less than 10 weight percent of titanium oxide.

11. The process according to any one of claims 1 to 10, wherein the supported catalyst exhibits at least one of the following features:
• a nitrogen BET specific area higher than or equal to 50 m²/g and lower than or equal to 400 m²/g;
• a nitrogen BET total pore volume higher than or equal to 0.2 cm³/g and lower than or equal to 1.5 cm³/g;
• a nitrogen BET average pore size higher than or equal to 2 nm and lower than or equal to 15 0 nm;
• a TEM average particle size of the first compound lower than or equal to 15 nm;
• a content of the at least one first compound with respect to the supported catalyst higher than or equal to 0.1 percent by weight and lower than or equal to 20 % by weight;
• and a content of the at least one second compound with respect to the supported catalyst higher than or equal to 1 percent by weight and lower than or equal to 30 percent by weight.

12. The process according to any one of claims 1 to 11, wherein the reaction is carried out in at least one of the following conditions:
• A temperature higher than or equal to 70 °C and lower than or equal to 300 °C;
• A hydrogen partial pressure higher than or equal to 1 bar absolute and lower than or equal to 200 bar absolute;
• A residence time of the liquid reaction medium higher than or equal to 5 min and lower than or equal to 25 h, when the process is carried out continuously.

13. The process according to any one of claims 1 to 12 carried out in the continuous mode.

14. A process for making a polyester comprising obtaining 1,3-propanediol according to the process of any one of claims 1 to 13, and further submitting said 1,3-propanediol to a reaction with a carboxylic acid and/or a carboxylic acid ester.

15. A process for making a polyester fiber comprising obtaining a polyester according to the process of claim 13, and further converting said polyester into a fiber.
